# FASCICULE DE BREVET EUROPEEN

(11) **EP 1 272 525 B1**
(45) Date de publication et mention de la délivrance du brevet: **16.08.2006**
(21) Numéro de dépôt: 01923782.5
(22) Date de dépôt: 10.04.2001
(51) Int. Cl.: C07K 16/30, G01N 33/574

(54) **ANTICORPS RECONNAISSANT SPECIFIQUEMENT LE PSA LIBRE INACTIF, ET LEURS APPLICATIONS**
ANTIKÖRPER, DIE SPEZIFISCH FÜR FREIES INAKTIVES PSA SIND, UND IHRE VERWENDUNGEN
NOVEL ANTIBODIES SPECIFICALLY IDENTIFYING UNBOUND INACTIVE PSA, AND USES THEREOF

(30) Priorité: 10.04.2000 FR 0004591
(43) Date de publication de la demande: 08.01.2003
(73) Titulaire: BIO MERIEUX, 69280 Marcy l'Etoile (FR)
(72) Inventeur: CHARRIER, Jean-Philippe, 69160 Tassin La Demi Lune (FR); JOLIVET-REYNAUD, Colette, F-69720 Saint Bonnet de Mure (FR); MICHEL, Sandrine, F-69001 Lyon (FR)
(74) Mandataire: Le Coupanec, Pascale A.M.P.
(86) Numéro de dépôt international: PCT/FR2001/001094
(87) Numéro de publication internationale: WO 2001/077180

(56) Documents cités:
- EP-A- 0 635 575
- WO-A-98/49323
- FR-A- 2 780 791
- US-A- 5 501 983
- US-A- 5 994 085
- MICHEL SANDRINE ET AL: "Anti-free prostate-specific antigen monoclonal antibody epitopes defined by mimotopes and molecular modeling." CLINICAL CHEMISTRY, vol. 45, no. 5, mai 1999 (1999-05), pages 638-650, XP002175112 ISSN: 0009-9147
- COREY EVA ET AL: "LNCaP produces both putative zymogen and inactive, free form of prostate-specific antigen." PROSTATE, vol. 35, no. 2, mai 1998 (1998-05), pages 135-143, XP000981985 ISSN: 0270-4137
- LAFFIN, ROBERT J. ET AL: "Hybritech total and free prostate-specific antigen assays developed for the Beckman Coulter Access automated chemiluminescent immunoassay system: a multicenter evaluation of analytical performance" CLIN. CHEM. (WASHINGTON, D. C.) ( 2001 ), 47(1), 129-132 , XP002175113
- MIKOLAJCZYK S D ET AL: 'A PRECURSOR FORM OF PROSTATE-SPECIFIC ANTIGEN IS MORE HIGHLY ELEVATED IN PROSTATE CANCER COMPARED WITH BEGIN TRANSITION ZONE PROSTATE TISSUE' CANCER RESEARCH vol. 60, pages 756 - 759, XP000939819

## Description

La présente invention se rapporte à des anticorps ou fragments d'anticorps qui se lient spécifiquement à l'antigène spécifique de la prostate (ou PSA, pour Prostate Specific Antigen) sous sa forme libre inactive, ainsi qu'aux hybridomes produisant de tels anticorps.

La présente invention se rapporte également à des réactifs pour tests immunologiques contenant de tels anticorps ainsi qu'à des tests immunologiques comprenant la mise en oeuvre de tels réactifs.

La présente invention se rapporte en outre à une méthode de diagnostic d'un adénocarcinome de la prostate chez un sujet soupçonné d'être atteint d'une telle pathologie.

La présente invention se rapporte également à une méthode de diagnostic permettant de différencier entre une pathologie bénigne de la prostate et un adénocarcinome de la prostate chez un sujet soupçonné d'être atteint d'une de ces maladies.

La présente invention se rapporte enfin au kit de diagnostic permettant de diagnostiquer un adénocarcinome de la prostate chez un sujet soupçonné d'être atteint d'un adénocarcinome ou permettant de différencier entre une pathologie bénigne de la prostate et un adénocarcinome de la prostate chez un sujet soupçonné d'être atteint par une de ces maladies.

Le PSA est produit par l'épithélium glandulaire de la prostate humaine, probablement sous une forme zymogène inactive (Lundwall *et al*. FEBS LeH 1987), et est sécrété dans le liquide séminal sous sa forme active (Lilja, J. Clin Invest 1985). L'activité biologique du PSA dans le liquide séminal est liée à sa fragmentation protéolytique limitée des protéines prédominantes sécrétées par les vésicules séminales (Lilja, J. Clin Invest 1985 ; Lilja *et al*. J. Clin Invest 1987 ; Mc Gee *et al*. Biol. reprod. 1988).

Le PSA est le principal marqueur du cancer de la prostate qui affectera, au cours de sa vie, un homme sur six en Occident. Cette protéase de la famille des kallikréines, principalement sécrétée par l'épithélium prostatique, est retrouvée à une concentration de 0,5 à 5 mg/ml dans le liquide séminal et à une concentration un million de fois moindre dans le sérum d'un patient. Ainsi, le PSA est trouvé normalement à une concentration inférieure à 2,5 ng/ml dans le sérum. Cependant, cette concentration augmente en principe notablement lors d'un cancer de la prostate et modérément lors d'altérations bénignes telles que l'hypertrophie de la prostate bénigne (BPH) ou la prostatite aiguë.

La séquence protéique du PSA a été déterminée. Il s'agit d'une glycoprotéine comportant 237 acides aminés ("Molecular cloning of human prostate specific antigen cDNA". Lundwall A., Lilja H., 1987. *FEBS Lett* 214 : 317-322).

Il a été proposé une méthode de diagnostic consistant à mesurer la concentration en PSA sérique et à la comparer à une valeur-seuil qui est de 4 ng/ml. Toutefois, on a constaté que cette méthode de diagnostic conduit à suspecter à tort trois patients sur quatre ce qui est préjudiciable. De plus, cette méthode ne permet pas de diagnostiquer 30 à 45% des cas de cancers confinés à la glande, ce qui constitue pourtant un stade précoce de la maladie potentiellement curable dont le diagnostic serait donc particulièrement souhaitable. Le caractère peu satisfaisant de cette méthode est d'ailleurs démontré par l'étude rapportée dans l'article "Prostate Cancer Detection in Men With Serum PSA Concentrations of 2.6 to 4.0 ng/ml and Benign Prostate Examination", Catalona *et al*., JAMA, 14 Mai 1997 - Vol. 277, No. 18, qui montre que la valeur-seuil doit être inférieure à 4 ng/ml pour dépister précocement un cancer de la prostate.

Par ailleurs, il a été montré récemment que dans le sérum, le PSA s'associait à des inhibiteurs de protéase, tels que l'α-1-antichymotrypsine (ACT), et l'α-2 macroglobuline (A2M). Ces associations entraînent l'inactivation de l'activité chymotrypsique du PSA, comme cela a été démontré dans l'article "Enzymatic activity of prostate specific antigen and its reactions with ultracellular serine proteinase inhibitors" A. Christensson, C.B. Laurell, H. Lilja, 1990 Eur. J. Biochem 194 : 755-763. L'usage du ratio PSA libre sur PSA total a alors été proposé afin d'améliorer la spécificité du diagnostic.

Ainsi, la demande de brevet WO-A-97/12245 décrit une méthode permettant de diagnostiquer un adénocarcinome de la prostate sans biopsie. Cette méthode consiste à mesurer, dans le sérum ou dans le sang des patients, la quantité totale de PSA Si cette valeur est comprise entre 2,5 et 20 ng/ml, on mesure également la concentration de PSA libre. On calcule ensuite le ratio PSA libre sur PSA total. Si ce ratio est inférieur à 7%, le diagnostic s'oriente vers un adénocarcinome de la prostate.

Toutefois, l'utilisation d'un seuil à 7%, pour le diagnostic d'un cancer de la prostate, est controversée par de nombreux auteurs, comme le montre la publication de Lein *et al.* "Relation of free PSA/total PSA in serum for differentiating between patients with prostatic cancer and benign prostate hyperplasia : which cutoff should be used ?". Dans ce document publié dans la revue Cancer Investigation, 16(1), 45-49, 1998, il a été démontré qu'il est difficile, par le biais de ce ratio, de différencier systématiquement un cancer de la prostate d'une BPH.

Pour ces raisons, la demanderesse s'est intéressée dans une demande de brevet WO-A-00/02052, déposée sous priorité du 3 Juillet 1998, à la présence dans le sérum de forme clivée du PSA. Les formes moléculaires de PSA sérique de patients atteints de cancer ou de BPH ont été cartographiées par électrophorèse bidimensionnelle, associée à ... une détection par chimioluminescence, afin d'observer l'ensemble des formes de PSA c'est-à-dire les formes libres, complexées et clivées.

Les profils d'électrophorèse de sérums de sujets atteints d'adénocarcinome de la prostate sont relativement homogènes, présentant essentiellement la forme non clivée du PSA, tandis que ceux de sujets atteints de BPH peuvent comporter une proportion relativement importante de forme clivée et des spots légèrement plus basiques sans forme clivée. L'augmentation du ratio PSA libre sérique sur PSA total sérique observé chez les patients atteints de BPH serait donc essentiellement liée à l'existence de PSA clivé, qui pourrait être enzymatiquement inactif et donc incapable de se lier à l'ACT ainsi qu'à la présence d'une forme de PSA libre légèrement plus basique que la forme PSA libre active, qui pourrait correspondre à la forme zymogène, inactive, du PSA.

En fonction de ces observations, la demanderesse a décrit des méthodes de diagnostic d'adénocarcinome de la prostate comprenant la quantification, après séparation par électrophorèse bidimensionnelle, du PSA libre clivé et/ou non clivé et l'utilisation de ces valeurs afin d'établir un diagnostic.

Toutefois, ces méthodes requièrent la mise en oeuvre d'électrophorèse bidimensionnelle. Elles sont par conséquent assez coûteuses et nécessitent beaucoup de temps de manipulation.

De WO 98/49323 sont connus des anticorps reconnaissant spécifiquement la forme zymogène du PSA et leur mise en oeuvre dans des méthodes de diagnostic du cancer de la prostate.

On a maintenant découvert qu'il était possible de disposer de méthodes de diagnostic d'adénocarcinomes de la prostate ne présentant pas les inconvénients précités et disposant d'une excellente spécificité et sensibilité par l'utilisation de réactifs immunologiques. On a en effet découvert qu'il est possible d'obtenir des anticorps dirigés spécifiquement contre le PSA libre inactif On a également découvert que de tels anticorps peuvent être avantageusement utilisés notamment dans des méthodes de diagnostic du cancer de la prostate et du BPH.

La présente demande a ainsi pour objet des anticorps ou fragments d'anticorps qui se lient spécifiquement au PSA libre inactif, tel que defini dans la revendication 1, lesdits anticorps étant des anticorps polyclonaux purifiés ou des anticorps monoclonaux.

Par fragments d'anticorps, on entend en général dans la présente demande, tout fragment d'anticorps ayant conservé la spécificité de l'anticorps d'origine et en particulier des fragments de type Fab et F(ab')₂. Dans la présente demande, le mot "anticorps" désigne par la suite également des fragments d'anticorps lorsque le sens le permet.

Par l'expression "anticorps se liant spécifiquement à une forme donnée de PSA", on désigne bien entendu un anticorps qui se lie essentiellement à ladite forme de PSA. Par exemple, le produit qui se trouve lié à l'anticorps est constitué d'au moins 80 %, et de préférence d'au moins 90 %, de ladite forme de PSA.

Par "PSA libre inactif", on entend dans la présente demande une forme libre du PSA, c'est-à-dire non complexée, autrement dit non associée à ses inhibiteurs tels que l'ACT. De plus, cette forme libre est inactive. Par le terme "inactif", on désigne une forme de PSA qui présente une activité protéolytique (en particulier de type chymiotrypsique) nettement inférieure à celle du PSA libre présent dans le sérum de sujets sains, par exemple inférieure d'au moins 80% ou davantage.

La présente invention a notamment pour objet des anticorps ou des fragments d'anticorps monoclonaux qui se lient spécifiquement au PSA libre inactif, tel que defini dans la revendication 1.

La présente invention a également pour objet les hybridomes produisant des anticorps monoclonaux tels que définis précédemment.

L'obtention d'hybridomes est bien connue et ne sera pas rappelée ici. On sélectionne les hybridomes qui reconnaissent spécifiquement le PSA libre inactif, tel que defini dans la revendication 1, comme indiqué ci-après. On a découvert que certains clones produisaient des anticorps qui, fixés sur une colonne de chromatographie, ne retenaient qu'une fraction du PSA total sérique de différents individus, et que cette fraction était, avec certains de ces anticorps, une forme libre et sensiblement inactive (telle que définie précédemment) du PSA.

Par ailleurs, on a constaté que les fractions de PSA non retenues par ces anticorps contenaient encore du PSA libre et avaient conservé pratiquement toute l'activité protéolytique du sérum de départ.

On a pu obtenir ainsi des fractions enrichies en PSA libre inactif qui ont servi ensuite à tester systématiquement des clones d'hybridomes obtenus comme précédemment.

Parmi les hybridomes obtenus après immunisation de souris avec du PSA d'origine séminale commercialisé par la société SCIPAC, on a constaté qu'environ 1,5% produisaient des anticorps se liant spécifiquement au PSA libre inactif ainsi obtenu.

La présente invention a également pour objet un réactif pour test immunologique contenant des anticorps ou fragments d'anticorps tels que définis précédemment.

Dans les réactifs selon l'invention, les anticorps ou fragments d'anticorps se liant spécifiquement au PSA libre inactif peuvent être utilisés tels quels ou bien notamment sous forme fixés sur un support solide et/ou liés à un marqueur.

La fixation d'anticorps ou de fragments d'anticorps sur un support solide est bien connue. Le support peut être réalisé avec tout matériau solide, biologique ou synthétique, doué de propriétés adsorbantes ou capables de fixer un agent de couplage. Des matériaux sont connus et décrits dans la littérature. Parmi les matériaux solides capables de fixer les anticorps par adsorption, on citera par exemple le polystyrène, le polypropylène, les latex, etc. Parmi les matériaux permettant de fixer les anticorps par covalence à l'aide d'un agent de couplage, on peut citer notamment le dextran, la cellulose, etc. Le support peut se présenter par exemple sous forme de disques, de tubes, de billes ou de plaques, en particulier de plaques de microtitrage.

La liaison d'un anticorps ou d'un fragment d'anticorps à un marqueur, qu'il soit radioactif, enzymatique, coloré ou de tout autre type communément utilisé dans des techniques immunologiques, est bien connue et décrite dans la littérature.

La présente invention a en outre pour objet un test immunologique pour la détermination quantitative du PSA libre inactif, tel que defini dans la revendication 1, dans un échantillon biologique prélevé chez un sujet, dans lequel on met en contact ledit échantillon avec un réactif tel que défini précédemment et l'on évalue la quantité de PSA libre inactif tel que defini dans la revendication 1, lié aux anticorps du réactif.

L'échantillon biologique prélevé chez le sujet, est généralement un échantillon de sang ou de sérum. Si désiré, il peut bien entendu avoir subi une étape de concentration ou de dilution préalablement à sa mise en contact avec le réactif contenant des anticorps ou fragments d'anticorps se liant spécifiquement au PSA libre inactif.

La quantification du PSA libre inactif lié auxdits anticorps ou fragments d'anticorps peut être réalisée de différentes manières bien connues.

Par exemple, la quantité de PSA libre inactif peut être évaluée par des tests de type sandwich. Selon un mode de réalisation particulier, on met en contact un réactif pour test immunologique, dans lequel les anticorps ou fragments d'anticorps se liant spécifiquement au PSA libre inactif sont fixés sur le support, avec l'échantillon biologique dont on souhaite déterminer la teneur en PSA libre inactif, puis après lavage l'on met en contact ledit support avec un second anticorps marqué se liant au PSA. Après un nouveau lavage, on mesure la quantité de marqueur fixé et on en déduit la teneur en PSA libre inactif par comparaison avec une courbe étalon établie préalablement.

Selon un autre mode de réalisation des tests immunologiques de l'invention, on évalue en outre la quantité d'une forme de PSA autre que la forme libre inactive présente dans un échantillon de même nature prélevé chez le même sujet.

Par l'expression "'échantillon de même nature prélevé chez le même sujet", on entend soit deux fractions d'un même prélèvement, soit deux échantillons issus de deux prélèvements différents mais qui doivent être de même nature, par exemple des échantillons sériques.

Bien entendu, lorsque les quantités mesurées sont destinées à être comparées, il convient que ces valeurs soient comparables. En d'autres termes, il convient que les valeurs mesurées soient rapportées à une même dilution ou concentration éventuelle de l'échantillon ainsi qu'à un même volume.

Les formes autres que la forme libre inactive du PSA sont notamment le PSA complexé, le PSA libre total, le PSA libre actif et le PSA total, c'est-à-dire l'ensemble des formes actives ou inactive, libres ou complexées, du PSA.

Le dosage de chacune de ces différentes formes notamment à l'aide d'anticorps spécifiques, est connu.

Le PSA complexé est dosé par exemple à l'aide d'anticorps décrits dans la demande de brevet WO-A-98/22509.

Le PSA total est dosé par exemple à l'aide d'anticorps décrits par H. Nagasaki et al. (1999), Clin. Chem. 45 : 4486-496.

Le PSA libre est dosé par exemple à l'aide d'anticorps décrits dans la demande de brevet WO 92/01936, ou à l'aide d'anticorps monoclonaux commercialisés par CHUGAI (Japon).

La présente invention a en particulier pour objet un test immunologique tel que défini précédemment dans lequel on dose en outre le PSA libre actif. La quantité de PSA libre actif peut être évaluée de différentes manières.

Ainsi, selon un premier mode de réalisation, la quantité de PSA libre actif est évaluée à l'aide d'anticorps se liant spécifiquement au PSA libre actif qui peuvent être obtenus par immunisation à l'aide de fractions de PSA total ou de fractions enrichies en PSA libre actif. La quantité de PSA libre actif peut être également évaluée de façon indirecte par évaluation de la quantité de PSA libre total, de laquelle on soustrait la quantité de PSA libre inactif déjà évaluée comme précédemment.

La présente invention a en outre pour objet un test immunologique pour déterminer le rapport de la quantité de PSA libre inactif tel que defini dans la revendication 1, à la quantité de PSA libre total,
ou le rapport de la quantité de PSA libre inactif tel que defini dans la revendication 1, à la quantité de PSA libre actif,
ou le rapport de la quantité de PSA libre actif à la quantité de PSA libre total,
ou le rapport de la quantité de PSA libre inactif tel que defini dans la revendication 1, à la quantité de PSA total,
ou le rapport de la quantité de PSA libre inactif tel que defini dans la revendication 1, à la quantité de PSA complexé,
ou le rapport de la quantité de PSA libre actif à la quantité de PSA total,
ou le rapport de la quantité de PSA libre actif à la quantité de PSA complexé,
ou les inverses de ces rapports, ou des combinaisons de ces rapports,
dans un échantillon biologique prélevé chez un sujet, dans lequel :
- on évalue la quantité de PSA libre inactif tel que defini dans la revendication 1, dans un echantillon biologique prélevé chez un sujet, en mettant en contact ledit échantillon avec un réactif selon l'invention tel que défini précédemment,
- on évalue l'une des quantités choisies parmi la quantité de PSA libre actif, la quantité de PSA libre total, la quantité de PSA total et la quantité de PSA complexé, sur un échantillon de même nature prélevé chez le même sujet, et
- on détermine ledit rapport ou ledit rapport inverse, ou ladite combinaison de rapports.

La présente invention concerne différentes méthodes de diagnostic qui, de façon générale, s'appliquent à des sujets présentant une concentration sérique en PSA supérieure à 2 ng/ml ou à 2,5 ng/ml.

La présente invention a également pour objet une méthode de diagnostic d'un adénocarcinome de la prostate chez un sujet soupçonné d'être atteint dudit adénocarcinome, sans pratiquer de biopsie, dans laquelle on met en oeuvre un ou plusieurs tests immunologiques tels que définis ci-dessus.

On a en effet découvert que la concentration (par exemple sérique) en PSA libre inactif est nettement plus élevée dans le cas d'une BPH que dans celui d'un adénocarcinome de la prostate.

L'invention concerne donc notamment une méthode de diagnostic de la BPH ou de différenciation entre un cancer de la prostate et une BPH, dans laquelle on mesure la teneur en PSA libre inactif, tel que defini dans la revendication 1, dans un échantillon provenant d'un individu, et on compare cette teneur à une échelle de valeurs prédéterminées, lesdites valeurs étant celles observées chez les patients affectés par une BPH reconnue et celles observées chez les patients affectés par un adénocarcinome de la prostate reconnu, et on conclut du résultat de cette comparaison soit à la présence d'une BPH soit à la présence d'un adénocarcinome de la prostate. Cette méthode revient en pratique à comparer la valeur mesurée à une valeur-seuil.

Bien entendu, ces variations de la teneur en PSA libre inactif influencent différents rapports tels que des rapports de la quantité de PSA libre inactif à la quantité de PSA libre total ou à la quantité de PSA total. On peut encore déterminer d'autres rapports, comme cela sera précisé ci-après.

On sait que, de façon générale, les résultats des tests immunologiques dépendent en grande partie des caractéristiques de spécificité et d'affinité des anticorps utilisés, et que ces caractéristiques influent sur les valeurs mesurées avec ces anticorps. On conçoit donc qu'il n'est pas possible de donner des valeurs-seuils précises et que des valeurs-seuils adaptées à chaque anticorps utilisé peuvent être déterminées dans chaque cas par de simples expériences de routine.

Il faut bien comprendre que l'on appelle ici valeur-seuil soit une valeur discrète soit un intervalle de valeurs correspondant à une zone d'indétermination. Bien évidemment, lorsque la valeur mesurée est incluse dans l'intervalle d'indétermination, ou est très proche de la valeur-seuil dans le cas d'une valeur discrète, on ne peut pas conclure définitivernent et il convient de conduire des investigations supplémentaires.

Bien entendu, quand on a déterminé une valeur-seuil pour un type de rapport donné, on peut en déduire des valeurs-seuils correspondant à d'autres types de rapports.

La présente invention a notamment pour objet une méthode de diagnostic telle que définie précédemment, dans laquelle, en outre, on compare la valeur du rapport ainsi déterminée à une valeur-seuil prédéterminée choisie, selon le type de rapport utilisé, de façon que les valeurs dudit rapport supérieures ou, respectivement, inférieures, à ladite valeur-seuil soient représentatives des valeurs observées chez les patients affectés par un adénocarcinome de la prostate reconnu, et de façon qu'inversement les valeurs dudit rapport inférieures ou, respectivement, supérieures, à ladite valeur-seuil soient représentatives des valeurs observées chez les sujets reconnus comme n'étant pas affectées par un adénocarcinome de la prostate, et l'on conclut du résultat de ladite comparaison soit à la présence soit à l'absence d'un adénocarcinome de la prostate.

La présente invention a également pour objet une méthode de diagnostic permettant de différencier entre une pathologie bénigne de la prostate et un adénocarcinome de la prostate, chez un sujet soupçonné d'être atteint par l'une de ces maladies dans laquelle on met en oeuvre un test immunologique tel que défini précédemment.

La présente invention a en particulier pour objet une telle méthode dans laquelle, en outre, on compare la valeur d'un des rapports mentionnés précédemment à une valeur de différenciation prédéterminée choisie, selon le type de rapport utilisé, de façon que les valeurs dudit rapport qui sont supérieures ou, respectivement, inférieures, à ladite valeur de différenciation soient représentatives des valeurs observées chez les patients affectés par une pathologie bénigne de la prostate reconnue, et de façon qu'inversement les valeurs dudit rapport qui sont inférieures ou, respectivement, supérieures, à ladite valeur de différenciation soient représentatives des valeurs observées chez des sujets reconnus comme étant affectés par l'adénocarcinome de la prostate reconnu, et l'on conclut du résultat de ladite comparaison soit à la présence d'une pathologie bénigne de la prostate soit à la présence d'un adénocarcinome de la prostate.

En pratique, les valeurs de différenciation, sont analogues aux valeurs-seuils discutées ci-dessus.

La présente invention a encore pour objet une méthode de diagnostic d'un adénocarcinome de la prostate chez un sujet soupçonné d'être atteint dudit adénocarcinome, dans laquelle :
- on détermine la quantité de PSA libre actif et la quantité de PSA libre inactif tel que defini dans la revendication 1, dans un échantillon prélevé chez ce sujet,
- le cas échéant, on détermine la quantité d'une forme de PSA autre que le PSA libre actif et autre que le PSA libre inactif tel que defini dans la revendication 1, dans un échantillon de même nature prélevé chez le même sujet, ladite autre forme étant telle que définie dans la revendication 18.
- on détermine la valeur de l'un des rapports suivants :
   - rapport de la quantité de PSA libre inactif tel que defini dans la revendication 1, à la quantité de PSA libre actif,
   - rapport de la quantité de PSA libre inactif tel que defini dans la revendication 1, à la quantité de PSA libre total,
   - rapport de la quantité de PSA libre inactiftel que defini dans la revendication 1, à la quantité de PSA total,
   - rapport de la quantité de PSA libre inactif tel que defini dans la revendication 1, à la quantité de PSA complexé,
ou les inverses de ces rapports, ou des combinaisons de ces rapports,
- on compare la valeur du rapport ainsi déterminée à une valeur-seuil prédéterminée choisie, selon le type de rapport utilisé, de façon que les valeurs dudit rapport supérieures ou, respectivement, inférieures, à ladite valeur-seuil soient représentatives des valeurs observées chez les patients affectés par un adénocarcinome de la prostate reconnu, et de façon qu'inversement les valeurs dudit rapport inférieures ou, respectiver ent, supérieures, à ladite valeur-seuil soient représentatives des valeu observées chez des sujets reconnus comme n'étant pas affectés par un adénocarcinome de la prostate et l'on conclut du résultat de ladite comparaison soit à la présence soit à l'absence d'un adénocarcinome de la prostate.

La présente invention a en outre pour objet une méthode de diagnostic permettant de différencier entre une pathologie bénigne de la prostate et un adénocarcinome de la prostate, chez un sujet soupçonné d'être atteint d'une de ces maladies, dans laquelle :
- on détermine la quantité de PSA libre actif et la quantité de PSA libre inactif tel que defini dans la revendication 1, dans un échantillon prélevé chez ce sujet,
- le cas échéant, on détermine la quantité d'une forme de PSA autre que le PSA libre actif et autre que le PSA libre inactif tel que defini dans la revendication 1, dans un échantillon de même nature prélevé chez le même sujet, ladite autre forme étant telle que définie dans la revendication 15.
- on détermine la valeur de l'un des rapports suivants :
   - rapport de la quantité de PSA libre inactif tel que defini dans la revendication 1, à la quantité de PSA libre actif,
   - rapport de la quantité de PSA libre inactif tel que defini dans la revendication 1, à la quantité de PSA libre total,
   - rapport de la quantité de PSA libre inactif tel que defini dans la revendication 1, à la quantité de PSA total,
   - rapport de la quantité de PSA libre inactif tel que defini dans la revendication 1, à la quantité de PSA complexé,
ou les inverses de ces rapports, ou des combinaisons de ces rapports,
- on compare la valeur du rapport ainsi déterminée à une valeur de différenciation prédéterminée choisie, selon le type de rapport utilisé, de façon que les valeurs dudit rapport sont supérieures ou, respectivement, inférieures, à ladite valeur de différenciation soient représentatives des valeurs observées chez les patients affectés par une pathologie bénigne de la prostate reconnue, et de façon qu'inversement les valeurs dudit rapport qui sont inférieures ou, respectivement, supérieures, à ladite valeur de différenciation soient représentatives des valeurs observées chez des sujets reconnus comme étant affectés par un adénocarcinome de la prostate reconnu, et l'on conclut du résultat de ladite comparaison soit à la présence d'une pathologie bénigne de la prostate soit à la présence d'un adénocarcinome de la prostate.

La présente invention a encore pour objet un kit de diagnostic permettant de diagnostiquer un adénocarcinome de la prostate chez un sujet soupçonné d'être atteint dudit adénocarcinome, ou permettant de différencier entre une pathologie bénigne de la prostate et un adénocarcinome de la prostate chez un sujet soupçonné d'être atteint par l'une de ces maladies, ledit kit comprenant :
- des anticorps conformes à l'invention pour doser le PSA libre inactif tel que defini dans la revendication 1, dans un échantillon biologique,
- des moyens pour doser le PSA libre actif dans un échantillon biologique de même nature, et éventuellement
- des moyens pour doser une forme de PSA autre que le PSA libre inactif , tel que defini dans la revendication 1, et autre que le PSA libre actif dans un échantillon biologique de même nature, ladite autre forme étant telle que définie dans la revendications 19.

Parmi les moyens utilisables dans ledit kit, on utilise en particulier des anti-corps se liant spécifiquement à la forme de PSA que l'on souhaite quantifier.

Les exemples suivants illustrent l'invention sans toutefois la limiter.

### Exemple 1 : Production et caractérisation d'anticorps monoclonaux

### Production d'anticorps monoclonaux se liant à la PSA

Des souris femelles BALB/c JYCo, âgées de 4 à 6 semaines (IFFA Credo) sont immunisées par injection intrapéritonéale avec 15 µg de PSA purifié (PSA d'origine séminale fournie par SCIPAC) émulsifiées avec un volume égal d'adjuvant complet de Freund, suivi par cinq injections d'adjuvant incomplet, les injections étant espacées de deux semaines. Quatre jours après la dernière injection, les cellules de la rate sont prélevées et fusionnées, selon la méthode de Köhler et Milstein, avec des cellules de la lignée cellulaire de myélome de souris Sp 2/0-Ag14. Puis les cellules sont mises en culture pendant 12 à 14 jours. Les surnageants des cultures sont récupérés puis soumis à un test selon une méthode ELISA dans laquelle la phase solide est revêtue avec l'antigène utilisé pour l'immunisation. Les colonies positives c'est-à-dire réagissant positivement vis-à-vis du PSA, sont sous-clonées deux fois par dilution limite. On cultive des clones sélectionnés en ascites de façon connue. La fraction IgG de chaque fluide ascitique est purifié par chromatographie sur colonne protéine A-Sépharose 4 FF, selon les instructions du fabricant (PHARMACIA).

### Exemple 2 : Détermination de la spécificité des anticorps monoclonaux vis-à-vis des formes libre, complexée et totale du PSA

Des plaques à 96 puits (Nunc) sont revêtues avec 100µl d'une solution comprenant 1 mg/L de PSA non complexé (fournie par SCIPAC), de complexe PSA-ACT (fourni par les Laboratoires SCRIPPS) ou d'ACT (également fourni par les Laboratoires SCRIPPS) dans 0,05 mol/L de tampon carbonate, PH 9,6. Après incubation pendant une nuit à température ambiante, les plaques sont lavées trois fois avec une solution saline tamponnée au phosphate (PBS ; 50 mmol/L tampon phosphate, PH 7,2, 150 mmol/L NaCl) contenant 0,5 ml/L de Tween 20 (PBS-T), et bloquée pendant 1 heure à 37°C avec du PBS contenant 10 g/L d'extrait de lait lyophilisé. Puis les plaques sont lavées une seconde fois avec du PBS-T. On ajoute dans chaque puits de chaque plaque 100 µl de fluide ascitique dilué de 10⁻¹ à 10⁻⁶ dans du PBS-T, puis on incube pendant une heure à 37°C. On lave ensuite les plaques avec du PBS-T, et on ajoute dans chaque puits de chaque plaque 100 µl d'IgG de chèvre anti-souris conjugué à de la phosphatase alcaline (Jackson ImunoResearch Laboratories) à une dilution de 1:2000 dans du PBS-T contenant 10 g/L d'albumine sérique bovine (BSA) ; (SIGMA). On incube les plaques pendant une heure à 37°C puis on les lave avec du PBS-T.

Le substrat, une solution de p-nitrophényl-phosphate (SIGMA) est ajoutée pendant 30 minutes. L'activité enzymatique est bloquée avec une solution de NaOH à 1 mol/L et l'absorbance est mesurée à 405 nm avec un lecteur de plaques ELISA (BioMérieux).

On a ainsi pu sélectionner plusieurs anticorps qui reconnaissent le PSA libre, mais qui ne reconnaissent ni le PSA complexé, ni l'ACT.

### Exemple 3 : Caractérisation de la spécificité vis-à-vis des formes inactive et active du PSA

Afin de poursuivre la caractérisation de la spécificité des anticorps, on a préparé du PSA présentant les caractéristiques souhaitées.

Ainsi, on fait synthétiser du PSA par la lignée cellulaire LNCaP selon la technique décrite dans les articles "LNCaP Produces both putative zymogen and inactive, free form of prostate specific antigen" E. Corey et al., 1998, Prostate 35:135-143, "Androgen-Sensitive human prostate cancer cell, LNCaP, Produce both N-terminaly mature a, d truncated prostate specific antigen isoform" A. Herrala et al., 1997, Eur. J. Biochem. 255:329-335, "Production of miligram concentration of free prostate specific antigen (fPSA) from LNCaP cell culture : Difference between fPSA from LNCaP cell and seminal plasma." J. T. Wu et al., 1998, J. Clin. Lab. Anal. 12 : 6-13.

Le PSA ainsi produit par la lignée cellulaire LNCaP est composé de PSA zymogène, de PSA mature enzymatiquement actif, de PSA entier inactif ainsi que de formes clivées ou tronquées inactives du PSA.

De plus, la culture de la lignée est réalisée en présence de sérum de veau foetal, qui contient de l'ACT, une proportion de complexe PSA-ACT étant ainsi également présente. Ce PSA d'origine cellulaire est donc un bon outil pour mettre en évidence la spécificité de reconnaissance des différents anticorps monoclonaux anti-PSA libre obtenus à l'exemple 2.

Des cellules LNCaP sont mises en culture dans un milieu synthétique auquel a été rajouté du sérum de veau foetal. Les différents surnageants de culture contenant le PSA sécrété, sont rassemblés, divisés en fractions identiques de 380 ml.

Des immunoabsorbants sont réalisés en couplant de façon identique des anticorps monoclonaux anti-PSA obtenus à l'exemple 2 sur résine de sépharose. On forme ainsi des colonnes d'affinité contenant des quantités de résine identiques. Chaque fraction de PSA cellulaire est immunopurifiée sur une de ces colonnes d'affinité, et le PSA fixé par chaque anticorps monoclonal est élué de la colonne, d'abord par une solution acide (glycine 0,1 M et pH 2,8), puis par une solution basique (DEA 0,1 M et pH 11). Les éluats sont ensuite immédiatement neutralisés pour préserver l'activité du PSA.

Le PSA contenu dans les différentes fractions obtenues est analysé : après avoir été quantifié, son activité enzymatique est mesurée. Puis les différentes formes de PSA contenues dans les éluats sont caractérisées par Western Blot après électrophorèse sur gel SDS-PAGE.

### (i) Quantité de PSA contenu dans les éluats

La quantité de PSA dans chaque fraction acide ou basique est déterminée par une méthode ELISA utilisant un anticorps polyclonal anti-PSA afin de s'assurer que le PSA sera dosé dans sa totalité.

Dans le tableau 1 ci-après, on donne les résultats obtenus avec 3 anticorps particuliers, spécifiques de la forme libre du PSA, obtenus à l'exemple 2.

**TABLEAU 1**

| *Dosage du PSA immunopurifié avec les anticorps monoclonaux anti-PSA (fractions "élutions acides" et "élutions basiques")* | | | | | | |
|---|---|---|---|---|---|---|
| | **ELUTION ACIDE** | | | **ELUTION BASIQUE** | | |
| **Anticorps monoclonaux** | **Concentration (µg/ml)** | **Volume fraction (ml)** | **Quantité PSA éluée (µg)** | **Concentration (µg/ml)** | **Volume fraction (ml)** | **Quantité PSA éluée (µg)** |
| **1** | 0,289 | 3,6 | 1,0 | 0,003 | 9,5 | 0,0 |
| **2** | 0,412 | 7,1 | 2,9 | 0,001 | 10,0 | 0,0 |
| **3** | 0,593 | 5,1 | 3,0 | 0,006 | 9,4 | 0,1 |
| **Surnageant cellulaire de départ** | 0,061 | 380,0 | 23,2 | | | |

Les résultats du tableau 1 montrent que le PSA est retrouvé exclusivement dans les fractions "éluats acides", et que tous les anticorps monoclonaux n'ont pas retenu la même quantité de PSA.

### (ii) Activité enzymatique spécifique du PSA purifié à l'aide ces anticorps

L'activité enzymatique du PSA contenu dans les fractions "élution acide" est mesurée sur un substrat de type chymotrypsine, S-2586 (MeO-Suc-Arg-Pro-Tyr-pNA.HCl) obtenu chez Chromogenix AB (Mölndal, Suède). Cette activité est déterminée sur 60 µl d'échantillon, et l'activité spécifique est calculée à partir de la concentration déterminée dans le paragraphe précédent.

Les résultats sont présentés dans le tableau 2 ci-dessous.

**TABLEAU 2**

| *Activité enzymatique spécifique du PSA cellulaire immunopurifié* | |
|---|---|
| Anticorps monoclonaux | Activité spécifique du PSA (Δ DO/min/µg de PSA x 1000) |
| 1 | 21,6 |
| 2 | 1,2 |
| 3 | 0,9 |
| Surnageant cellulaire de départ | 11,6 |

Les résultats montrent que l'activité enzymatique spécifique de l'éluat acide obtenu avec l'anticorps anti-PSA n° 1 est deux fois plus élevée que l'activité du surnageant cellulaire de départ, avant immunopurification.

Cet anticorps a donc enrichi le PSA purifié en forme enzymatiquement active. De plus, cet anticorps n° 1 reconnaît une forme libre du PSA. Par conséquent, l'anticorps n° 1 reconnaît essentiellement la forme libre active du PSA.

L'activité enzymatique spécifique des éluats acides obtenus avec les anticorps n° 2 et 3, est très faible. Cela montre que ces anticorps ont retenu essentiellement du PSA sous sa forme inactive. Comme ces anticorps sont spécifiques d'une forme libre du PSA, ils reconnaissent essentiellement le PSA sous sa forme libre inactive.

De plus, on sait que la trypsine est capable de transformer le PSA zymogène en PSA enzymatiquement actif C'est pourquoi, afin de déterminer si le PSA zymogène avait été retenu, lors des opérations de chromatographie d'affinité décrites ci-dessus, par les anticorps monoclonaux anti-PSA, l'activité enzymatique des "éluats acides" a été mesurée en présence de trypsine. L'activité spécifique des éluats, déterminée avec ou sans trypsine, est identique. Par conséquent, les éluats acides obtenus avec les anticorps n° 1, 2 et 3, comme décrit ci-dessus, ne contiennent pas de PSA sous forme zymogène.

Donc aucun des anticorps 1, 2 ou 3 ne reconnaît la forme zymogène du PSA.

### 2) Immunoanalyse effectuée après électrophorèse sur gel SDS-PAGE :

Les différentes formes de PSA présentes dans les éluats issus des colonnes sur lesquelles sont fixés les anticorps n° 1, 2 et 3 ont été séparées par électrophorèse sur gel d'acrylamide en conditions réductrices, et la détection du PSA par Western Blot est réalisée avec un anticorps monoclonal reconnaissant le PSA total.

Les Western Blot réalisés confirment que du PSA est présent dans tous les échantillons testés provenant de l'élution acide, dans des proportions en corrélation avec les quantités déterminées dans le tableau 1, alors que les échantillons provenant des élutions basiques n'en contiennent pas.

Les Western Blot réalisés confirment également que les anticorps n° 1, 2 et 3 n'ont pas retenu le complexe PSA-ACT. Enfin, par comparaison avec le PSA obtenu comme indiqué à l'exemple 3 utilisé comme témoin, les élutions acides contiennent des formes de PSA ayant un poids moléculaire normal (33 kDa), et également des formes ayant un poids moléculaire inférieur.

## Revendications

1. Anticorps ou fragments d'anticorps qui se lient spécifiquement au PSA libre inactif, ledit PSA libre inactif étant constitué de PSA libre inactif clivé et de la forme zymogène du PSA, lesdits anticorps étant des anticorps polyclonaux purifiés ou des anticorps monoclonaux.

2. Anticorps ou fragments d'anticorps selon la revendication 1, qui sont des anticorps monoclonaux.

3. Hybridome produisant des anticorps monoclonaux tels que définis dans la revendication 2.

4. Réactif pour test immunologique contenant des anticorps ou fragments d'anticorps tels que définis dans la revendication 1 ou 2.

5. Réactif pour test immunologique tel que défini dans la revendication 4, dans lequel lesdits anticorps ou fragments d'anticorps sont fixés sur un support et/ou sont liés à un marqueur.

6. Test immunologique pour la détermination quantitative du PSA libre inactif, ledit PSA libre inactif étant constitué de PSA libre inactif clivé et de la forme zymogène du PSA dans un échantillon biologique prélevé chez un sujet, dans lequel on met en contact ledit échantillon avec un réactif tel que défini dans la revendication 4 ou 5, et l'on évalue la quantité de PSA libre inactif, ledit PSA libre inactif étant constitué de PSA libre inactif clivé et de la forme zymogène du PSA lié auxdits anticorps ou fragments d'anticorps.

7. Test immunologique selon la revendication 6, dans lequel, en outre, on évalue la quantité d'une forme de PSA autre que la forme libre inactive présente dans un échantillon de même nature prélevé chez le même sujet.

8. Test immunologique selon la revendication 7, dans lequel ladite autre forme de PSA est choisie parmi le PSA libre actif, le PSA libre total, le PSA complexé et le PSA total.

9. Test immunologique selon l'une quelconque des revendications 7 à 8 dans lequel, en outre, on détermine au moins un rapport de la quantité de PSA libre inactif, ledit PSA libre inactif étant constitué de PSA libre inactif clivé et de la forme zymogène du PSA à la quantité de ladite autre forme, ou les inverses de ces rapports, ou des combinaisons de ces rapports.

10. Test immunologique selon la revendication 9, dans lequel, en outre, on détermine au moins un rapport choisi parmi le rapport de la quantité de PSA libre actif à la quantité de PSA libre total, ou le rapport de la quantité de PSA libre actif à la quantité de PSA total, ou le rapport de la quantité de PSA libre actif à la quantité de PSA complexé, ou les inverses de ces rapports, ou des combinaisons de ces rapports.

11. Test immunologique selon les revendications 8 à 10, dans lequel ladite autre forme est le PSA libre actif.

12. Méthode de diagnostic d'une pathologie bénigne de la prostate ou de différenciation entre une pathologie bénigne de la prostate et un adénocarcinome de la prostate chez un sujet, dans laquelle on mesure la teneur en PSA libre inactif, ledit PSA libre inactif étant constitué de PSA libre inactif clivé et de la forme zymogène du PSA dans un échantillon provenant dudit sujet au moyen d'un test immunologique tel que défini dans les revendications 6 à 11.

13. Méthode selon la revendications 12, dans laquelle, en outre, on compare ladite teneur à une échelle de valeurs prédéterminées, lesdites valeurs étant celles observées chez les patients affectés par une pathologie bénigne de la prostate reconnue et celles observées chez les patients affectés par un adénocarcinome de la prostate reconnu, et l'on conclut du résultat de cette comparaison soit à la présence d'une pathologie bénigne de la prostate soit à la présence d'un adénocarcinome de la prostate.

14. Méthode selon la revendications 12 ou 13, dans laquelle, en outre, on compare la valeur du rapport ainsi déterminée à une valeur de différenciation prédéterminée choisie, selon le type de rapport utilisé, de façon que les valeurs dudit rapport qui sont supérieures ou, respectivement, inférieures, à ladite valeur de différenciation soient représentatives des valeurs observées chez les patients affectés par une pathologie bénigne de la prostate reconnue, et de façon qu'inversement les valeurs dudit rapport qui sont inférieures ou, respectivement, supérieures, à ladite valeur de différenciation soient représentatives des valeurs observées chez des sujets reconnus comme étant affectés par un adénocarcinome de la prostate reconnu, et l'on conclut du résultat de ladite comparaison soit à la présence d'une pathologie bénigne de la prostate soit à la présence d'un adénocarcinome de la prostate.

15. Méthode selon l'une quelconque des revendications 12 à 14, dans laquelle :
- on détermine la quantité de PSA libre actif et la quantité de PSA libre inactif, ledit PSA libre inactif étant constitué de PSA libre inactif clivé et de la forme zymogène du PSA dans un échantillon prélevé chez ce sujet,
- le cas échéant, on détermine la quantité d'une forme de PSA autre que le PSA libre actif et autre que le PSA libre inactif, ledit PSA libre inactif étant constitué de PSA libre inactif clivé et de la forme zymogène du PSA dans un échantillon de même nature prélevé chez le même sujet, ladite forme étant choisie parmi le PSA libre total, le PSA total et le PSA complexé,
- on détermine la valeur de l'un des rapports suivants :
• rapport de la quantité de PSA libre inactif, ledit PSA libre inactif étant constitué de PSA libre inactif clivé et de la forme zymogène du PSA à la quantité de PSA libre actif,
• rapport de la quantité de PSA libre inactif, ledit PSA libre inactif étant constitué de PSA libre inactif clivé et de la forme zymogène du PSA à la quantité de PSA libre total,
• rapport de la quantité de PSA libre inactif, ledit PSA libre inactif étant constitué de PSA libre inactif clivé et de la forme zymogène du PSA à la quantité de PSA total,
• rapport de la quantité de PSA libre inactif, ledit PSA libre inactif étant constitué de PSA libre inactif clivé et de la forme zymogène du PSA à la quantité de PSA complexé.
ou les inverses de ces rapports, ou des combinaisons de ces rapports,
- on compare la valeur du rapport ainsi déterminée à une valeur de différenciation prédéterminée choisie, selon le type de rapport utilisé, de façon que les valeurs dudit rapport qui sont supérieures ou, respectivement, inférieures, à ladite valeur de différenciation soient représentatives des valeurs observées chez les patients affectés par une pathologie bénigne de la prostate reconnue, et de façon qu'inversement les valeurs dudit rapport qui sont inférieures ou, respectivement, supérieures, à ladite valeur de différenciation soient représentatives des valeurs observées chez des sujets reconnus comme étant affectés par un adénocarcinome de la prostate reconnu, et l'on conclut du résultat de ladite comparaison soit à la présence d'une pathologie bénigne de la prostate soit à la présence d'un adénocarcinome de la prostate.

16. Méthode de diagnostic d'un adénocarcinome de la prostate chez un sujet soupçonné d'être atteint dudit adénocarcinome, sans pratiquer de biopsie, dans laquelle on met en oeuvre un test immunologique tel que défini selon les revendications 6 à 11.

17. Méthode selon la revendication 16, dans laquelle, en outre, on compare la valeur du rapport ainsi déterminée à une valeur-seuil prédéterminée choisie, selon le type de rapport utilisé, de façon que les valeurs dudit rapport supérieures ou, respectivement, inférieures, à ladite valeur-seuil soient représentatives des valeurs observées chez les patients affectés par un adénocarcinome de la prostate reconnu, et de façon qu'inversement les valeurs dudit rapport inférieures ou, respectivement, supérieures, à ladite valeur-seuil soient représentatives des valeurs observées chez des sujets reconnus comme n'étant pas affectés par un adénocarcinome de la prostate, et l'on conclut du résultat de ladite comparaison soit à la présence soit à l'absence d'un adénocarcinome de la prostate.

18. Méthode selon la revendication 16 ou 17, dans laquelle :
- on détermine la quantité de PSA libre actif et la quantité de PSA libre inactif, ledit PSA libre inactif étant constitué de PSA libre inactif clivé et de la forme zymogène du PSA dans un échantillon prélevé chez ce sujet,
- le cas échéant, on détermine la quantité d'une forme de PSA autre que le PSA libre actif et autre que le PSA libre inactif, ledit PSA Gbre inactif étant constitué de PSA libre inactif clivé et de la forme zymogène du PSA dans un échantillon de même nature prélevé chez le même sujet, choisie parmi le PSA libre total, le PSA total, le PSA complexé,
- on détermine la valeur de l'un des rapports suivants :
• rapport de la quantité de PSA libre inactif, ledit PSA libre inactif étant constitué de PSA libre inactif clivé et de la forme zymogène du PSA à la quantité de PSA libre actif,
• rapport de la quantité de PSA libre inactif, ledit PSA libre inactif étant constitué de PSA libre inactif clivé et de la forme zymogène du PSA à la quantité de PSA libre total,
• rapport de la quantité de PSA libre inactif, ledit PSA libre inactif étant constitué de PSA libre inactif clivé et de la forme zymogène du PSA à la quantité de PSA total,
• rapport de la quantité de PSA libre inactif, ledit PSA libre inactif étant constitué de PSA libre inactif clivé et de la forme zymogène du PSA à la quantité de PSA complexé,
ou les inverses de ces rapports, ou des combinaisons de ces rapports,
- on compare la valeur du rapport ainsi déterminée à une valeur-seuil prédéterminée choisie, selon le type de rapport utilisé, de façon que les valeurs dudit rapport supérieures ou, respectivement, inférieures, à ladite valeur-seuil soient représentatives des valeurs observées chez les patients affectés par un adénocarcinome de la prostate reconnu, et de façon qu'inversement les valeurs dudit rapport inférieures ou, respectivement, supérieures, à ladite valeur-seuil soient représentatives des valeurs observées chez des sujets reconnus comme n'étant pas affectés par un adénocarcinome de la prostate, et l'on conclut du résultat de ladite comparaison soit à la présence soit à l'absence d'un adénocarcinome de la prostate.

19. Kit de diagnostic permettant de diagnostiquer un adénocarcinome de la prostate chez un sujet soupçonné d'être atteint dudit adénocarcinome, ou permettant de différencier entre une pathologie bénigne de la prostate et un adénocarcinome de la prostate chez un sujet soupçonné d'être atteint par l'une de ces maladies, ledit kit comprenant :
- des anticorps tels que definis selon la revendication 1 ou 2 pour doser le PSA libre inactif, ledit PSA libre inactif étant constitué de PSA libre inactif clivé et de la forme zymogène du PSA dans un échantillon biologique, et/ou
- des moyens pour doser le PSA libre actif dans un échantillon biologique de même nature, et éventuellement
- des moyens pour doser une forme de PSA autre que le PSA libre inactif, ledit PSA libre inactif étant constitué de PSA libre inactif clivé et de la forme zymogène du PSA et autre que le PSA libre actif dans un échantillon biologique de même nature, choisie parmi le PSA libre total, le PSA total et le PSA complexé.

20. Kit selon la revendication 19, dans lequel lesdits moyens sont des anticorps.

## Claims

1. An antibody or antibody fragment which binds specifically to inactive free PSA, said inactive free PSA being constituted of cleaved inactive free PSA and of the zymogen form of PSA, said antibody being a purified polyclonal antibody or a monoclonal antibody.

2. The antibody or antibody fragment as claimed in claim 1, which is a monoclonal antibody.

3. A hybridoma which produces monoclonal antibodies as defined in claim 2.

4. A reagent for immunoassay containing antibodies or antibody fragments as defined in claim 1 or 2.

5. The reagent for immunoassay as defined in claim 4, in which said antibodies or antibody fragments are attached to a support and/or are linked to a label.

6. An immunoassay for the quantitative determination of inactive free PSA, said inactive free PSA being constituted of cleaved inactive free PSA and of the zymogen form of PSA, in a biological sample taken from an individual, in which said sample is brought into contact with a reagent as defined in claim 4 or 5, and the amount of inactive free PSA, said inactive free PSA being constituted of cleaved inactive free PSA and of the zymogen form of PSA bound to said antibodies or antibody fragments is evaluated.

7. The immunoassay as claimed in claim 6, in which, in addition, the amount of a form of PSA other than the inactive free form, present in a sample of the same nature taken from the same individual, is evaluated.

8. The immunoassay as claimed in claim 7, in which said other form of PSA is chosen from active free PSA, total free PSA, complexed PSA and total PSA.

9. The immunoassay as claimed in claim 7 or 8, in which, in addition, a ratio of the amount of inactive free PSA, said inactive free PSA being constituted of cleaved inactive free PSA and of the zymogen form of PSA, to the amount of said other form, or said inverse ratio or said combination of ratios is determined.

10. The immunoassay as claimed in claim 9, in which, in addition, a ratio chosen from the ratio of the amount of active free PSA to the amount of total free PSA, or the ratio of the amount of active PSA to the amount of complexed PSA, or said inverse ratio or said combination of ratios is determined.

11. The immunoassay as claimed in claims 8 to 10, in which said other form is the active free PSA.

12. A method for diagnosing a benign pathology of the prostate or for differentiating between a benign pathology of the prostate and an adenocarcinoma of the prostate in an individual, in which the inactive free PSA content, said inactive free PSA being constituted of cleaved inactive free PSA and of the zymogen form of PSA, in a sample originating from said individual is measured by means of an immunoassay as defined according to claims 6 to 11.

13. The method as claimed in claim 12, in which, in addition, said content is compared with a scale of predetermined values, said values being those observed in patients suffering from a recognized benign pathology of the prostate and those observed in patients suffering from a recognized adenocarcinoma of the prostate, and it is concluded, from the result of this comparison, either that there is a benign pathology of the prostate or that there is an adenocarcinoma of the prostate.

14. The method as claimed in claim 12 or 13, in which, in addition, the value of the ratio thus determined is compared with a predetermined differentiation value chosen, depending on the type of ratio used, such that the values of said ratio higher or, respectively, lower, than said differentiation value are representative of the values observed in patients suffering from a recognized benign pathology of the prostate, and such that, conversely, the values of said ratio lower or, respectively, higher, than said differentiation value are representative of the values observed in individuals recognized as suffering from a recognized adenocarcinoma of the prostate, and it is concluded, from the result of said comparison, that there is either a benign pathology of the prostate, or an adenocarcinoma of the prostate.

15. A method according to any one of claims 12 to 14, in which:
- the amount of active free PSA and the amount of inactive free PSA, said inactive free PSA being constituted of cleaved inactive free PSA and of the zymogen form of PSA in a sample taken from this individual is determined,
- where appropriate, the amount of a form of PSA other than active free PSA and other than inactive free PSA, said inactive free PSA being constituted of cleaved inactive free PSA and of the zymogen form of PSA in a sample of the same nature taken from the same individual, is determined, said form being chosen from total free PSA, total PSA and complexed PSA,
- the value of one of the following ratios is determined:
• the ratio of the amount of inactive free PSA, said inactive free PSA being constituted of cleaved inactive free PSA and of the zymogen form of PSA to the amount of active free PSA,
• the ratio of the amount of inactive free PSA, said inactive free PSA being constituted of cleaved inactive free PSA and of the zymogen form of PSA to the amount of total free PSA,
• the ratio of the amount of inactive free PSA, said inactive free PSA being constituted of cleaved inactive free PSA and of the zymogen form of PSA to the amount of total PSA,
• the ratio of the amount of inactive free PSA, said inactive free PSA being constituted of cleaved inactive free PSA and of the zymogen form of PSA to the amount of complexed PSA,
or the inverses of these ratios, or combinations of these ratios,
- the value of the ratio thus determined is compared with a predetermined differentiation value chosen, depending on the type of ratio used, such that the values of said ratio higher or, respectively, lower, than said differentiation value are representative of the values observed in patients suffering from a recognized benign pathology of the prostate, and such that, conversely, the values of said ratio lower or, respectively, higher, than said differentiation value are representative of the values observed in individuals recognized to be suffering from a recognized adenocarcinoma of the prostate, and it is concluded, from the result of said comparison that there is either a benign pathology of the prostate or an adenocarcinoma of the prostate.

16. A diagnostic method of an adenocarcinoma of the prostate in an individual suspected of suffering from said adenocarcinoma without performing a biopsy, in which an immunoassay as defined according to claims 6 to 11 is carried out.

17. The method as claimed in claim 16, in which, in addition, the value of the ratio thus determined is compared with a predetermined treshold value chosen, depending on the type of ratio used, such that the values of said ratio which are higher or, respectively, lower, than said treshold value are representative of the values observed in patients suffering from a recognized adenocarcinoma of the prostate, and such that, conversely, the values of said ratio which are lower or, respectively, higher, than said treshold value are representative of the values observed in individuals recognized not to be suffering from a recognized adenocarcinoma of the prostate, and it is concluded, from the result of said comparison, either that an adenocarcinoma of the prostate is present or that one is not present.

18. The method according to claim 16 or 17, in which:
- the amount of active free PSA and the amount of inactive free PSA, said inactive free PSA being constituted of cleaved inactive free PSA and of the zymogen form of PSA, in a sample taken from this individual is determined,
- where appropriate, the amount of a form of PSA other than active free PSA and other than inactive free PSA, said inactive free PSA being constituted of cleaved inactive free PSA and of the zymogen form of PSA, in a sample of the same nature taken from the same individual, is determined, chosen from total free PSA, total PSA complexed PSA,
- the value of one of the following ratios is determined:
• ratio of the amount of inactive free PSA, said inactive free PSA being constituted of cleaved inactive free PSA and of the zymogen form of PSA, to the amount of active free PSA,
• ratio of the amount of inactive free PSA, said inactive free PSA being constituted of cleaved inactive free PSA and of the zymogen form of PSA, to the amount of total free PSA,
• ratio of the amount of inactive free PSA, said inactive free PSA being constituted of cleaved inactive free PSA and of the zymogen form of PSA, to the amount of total PSA,
• ratio of the amount of inactive free PSA, said inactive free PSA being constituted of cleaved inactive free PSA and of the zymogen form of PSA, to the amount of complexed PSA,
or the inverses of these ratios, or combinations of these ratios,
- the value of the ratio thus determined is compared with a predetermined threshold value chosen, depending on the type of ratio used, such that the values of said ratio higher or, respectively, lower, than said threshold value are representative of the values observed in patients suffering from a recognized adenocarcinoma of the prostate, and such that, conversely, the values of said ratio lower or, respectively, higher, than said threshold value are representative of the values observed in individuals recognized not to be suffering from an adenocarcinoma of the prostate, and it is concluded, from the result of said comparison, either that an adenocarcinoma of the prostate is present, or that one is not present.

19. A diagnostic kit for diagnosing an adenocarcinoma of the prostate in an individual suspected of suffering from said adenocarcinoma, or for differentiating between a benign pathology of the prostate and an adenocarcinoma of the prostate in an individual suspected of suffering from one of these diseases, said kit comprising:
- antibodies as defined according to claim 1 or 2 for assaying inactive free PSA, said inactive free PSA being constituted of cleaved inactive free PSA and of the zymogen form of PSA, in a biological sample, and/or
- means for assaying active free PSA in a biological sample of the same nature and, optionally,
- means for assaying a form of PSA other than inactive free PSA, said inactive free PSA being constituted of cleaved inactive free PSA and of the zymogen form of PSA, and other than active free PSA, in a biological sample of the same nature, chosen from total free PSA, total PSA and complexed PSA.

20. The kit as claimed in claim 19, in which said means are antibodies.

## Patentansprüche

1. Antikörper oder Antikörperfragmente, die sich spezifisch an das inaktive freie PSA binden, wobei dieses inaktive reie PSA aus gespaltenem inaktiven freien PSA und aus der Zymogenform des PSA besteht, wobei diese Antikörper gereinigte polyklonale Antikörper oder monoklonale Antikörper sind.

2. Antikörper oder Antikörperfragmente nach Anspruch 1, die monoklonale Antikörper sind.

3. Hybridom, das monoklonale Antikörper erzeugt, wie sie in Anspruch 2 definiert sind.

4. Reagens für einen immunologischen Test, enthaltend Antikörper oder Antikörperfragmente, wie sie in Anspruch 1 oder 2 definiert sind.

5. Reagens für einen immunologischen Test, wie es in Anspruch 4 definiert ist, bei dem die Antikörper oder die Antikörperfragmente an einem Träger fixiert sind und/oder an einen Marker gebunden sind.

6. Immunologischer Test für die quantitative Bestimmung des inaktiven freien PSA, wobei dieses inaktive freie PSA aus gespaltenem inaktiven freien PSA und der Zymogenform des PSA besteht, in einer bei einem Patienten entnommenen biologischen Probe, bei dem man diese Probe mit einem Reagens, wie es in Anspruch 4 oder 5 definiert ist, in Kontakt bringt und die Menge an inaktivem freien PSA ermittelt, wobei dieses inaktive freie PSA aus gespaltenem inaktiven freien PSA und der Zymogenform des PSA besteht, das an die Antikörper oder Antikörperfragmente gebunden ist.

7. Immunologischer Text nach Anspruch 6, bei dem man außerdem die Menge einer anderen PSA-Form als der inaktiven freien Form ermittelt, die in einer bei demselben Patienten entnommenen Probe von derselben Natur vorliegt.

8. Immunologischer Test nach Anspruch 7, bei dem diese andere PSA-Form aus dem aktiven freien PSA, dem gesamten freien PSA, dem komplexierten PSA und dem Gesamt-PSA ausgewählt ist.

9. Immunologischer Test nach einem der Ansprüche 7 bis 8, bei dem man außerdem mindestens ein Verhältnis der Menge an inaktivem freien PSA, wobei dieses inaktive freie PSA aus gespaltenem inaktiven freien PSA und der Zymogenform des PSA besteht, zur Menge dieser anderen Form oder die Kehrwerte dieser Verhältnisse oder Kombinationen dieser Verhältnisse bestimmt.

10. Immunologischer Test nach Anspruch 9, bei dem man außerdem mindestens ein Verhältnis bestimmt, das aus dem Verhältnis der Menge an aktivem freien PSA zur Menge an gesamtem freien PSA, oder dem Verhältnis der Menge an aktivem freien PSA zur Menge an Gesamt-PSA oder dem Verhältnis der Menge an aktivem freien PSA zur Menge an komplexiertem PSA ausgewählt ist, oder die Kehrwerte dieser Verhältnisse oder Kombinationen dieser Verhältnisse.

11. Immunologischer Test nach Anspruch 8 bis 10, bei dem diese andere Form das aktive freie PSA ist.

12. Methode zur Diagnostik einer gutartigen Prostataerkrankung oder zur Differenzierung zwischen einer gutartigen Prostataerkrankung und einem Prostata-Adenokarzinom bei einem Patienten, bei der man den Gehalt an inaktivem freien PSA, wobei dieses inaktive freie PSA aus gespaltenem inaktiven freien PSA und der Zymogenform des PSA besteht, in einer von diesem Patienten stammenden Probe mittels eines immunologischen Tests misst, wie er in den Ansprüche 6 bis 11 definiert ist.

13. Methode nach Anspruch 12, bei der man außerdem diesen Gehalt mit einer Skala von vorbestimmten Werten vergleicht, wobei diese Werte diejenigen sind, die bei Patienten mit einer anerkannten gutartigen Prostataerkrankung beobachtet wurden, und diejenigen, die bei Patienten mit einem anerkannten Prostata-Adenokarzinom beobachtet wurden, und man aus dem Ergebnis dieses Vergleichs entweder auf das Vorliegen einer gutartigen Prostataerkrankung oder auf das Vorliegen eines Prostata-Adenokarzinoms schließt.

14. Methode nach Anspruch 12 oder 13, bei der man außerdem den auf diese Weise bestimmten Wert des Verhältnisses mit einem gewählten vorbestimmten Differenzierungswert vergleicht, der je nach dem verwendeten Verhältnistyp gewählt ist, so dass die Werte dieses Verhältnisses, die größer bzw. kleiner als dieser Differenzierungswert sind, repräsentativ für die Werte sind, die bei Patienten mit einer anerkannten gutartigen Prostataerkrankung beobachtet wurden, und so dass umgekehrt die Werte dieses Verhältnisses, die kleiner bzw. größer als dieser Differenzierungswert sind, repräsentativ für die Werte sind, die bei Patienten beobachtet wurden, die dafür anerkannt sind, dass sie an einem anerkannten Prostata-Adenokarzinom leiden, und man aus dem Ergebnis dieses Vergleichs entweder auf das Vorliegen einer gutartigen Prostataerkrankung oder auf das Vorliegen eines Prostata-Adenokarzinoms schließt.

15. Methode nach einem der Ansprüche 12 bis 14, bei der man:
- die Menge an aktivem freien PSA und die Menge an unaktivem freien PSA, wobei das inaktive freie PSA aus gespaltenem inaktiven freien PSA und der Zymmogenform des PSA besteht, in einer bei diesem Patienten entnommenen Probe bestimmt,
- ggf. die Menge einer anderen PSA-Form als dem aktiven freien PSA und dem inaktiven freien PSA, wobei dieses inaktive freie PSA aus gespaltenem inaktiven freien PSA und der Zymogenform des PSA besteht, in einer bei demselben Patienten entnommenen Probe derselben Natur bestimmt, wobei diese Form aus gesamtem freien PSA, Gesamt-PSA und komplexiertem PSA ausgewählt ist,
den Wert eines der folgenden Verhältnisse bestimmt:
- Verhältnis der Menge an inaktivem freien PSA, wobei dieses inaktive freie PSA aus gespaltenem inaktiven freien PSA und der Zymogenform des PSA besteht, zur Menge an aktivem freien PSA,
Verhältnis der Menge an inaktivem freien PSA, wobei dieses inaktive freie PSA aus gespaltenem inaktiven freien PSA und der Zymogenform des PSA besteht, zur Menge an gesamtem freien PSA,
Verhältnis der Menge an inaktivem freien PSA, wobei dieses inaktive freie PSA aus gespaltenem inaktiven freien PSA und der Zymogenform des PSA besteht, mit der Menge an Gesamt-PSA,
Verhältnis von inaktivem freien PSA, wobei dieses inaktive freie PSA aus gespaltenem inaktiven freien PSA und der Zymogenform des PSA besteht, zur Menge an komplexiertem PSA,
oder die Kehrwerte dieser Verhältnisse oder Kombinationen dieser Verhältnisse,
- den auf diese Weise bestimmten Wert des Verhältnisses mit einem vorbestimmten Differenzierungswert vergleicht, der je nach dem verwendeten Verhältnistyp gewählt ist, so dass die Werte dieses Verhältnisses, die größer bzw. kleiner als der Differenzierungswert sind, repräsentativ für die Werte sind, die bei Patienten mit einer anerkannten gutartigen Prostataerkrankung beobachtet wurden, und so dass umgekehrt die Werte dieses Verhältnisses, die kleiner bzw. größer als dieser Differenzierungswert sind, repräsentativ für die Werte sind, die bei Patienten beobachtet wurden, die dafür anerkannt sind, dass sie an einem anerkannten Prostata-Adenokarzinom leiden, und man aus dem Ergebnis dieses Vergleichs entweder auf das Vorliegen einer gutartigen Prostataerkrankung oder auf das Vorliegen eines Prostata-Adenokarzinoms schließt.

16. Methode zur Diagnostik eines Prostata-Karzinoms bei einem Patienten mit einem Verdacht auf dieses Adenokarzinom ohne Durchführung einer Biopsie, bei der man einen immunologischen Test ausführt, wie er in den Ansprüchen 6 bis 11 definiert ist.

17. Methode nach Anspruch 16, bei der man außerdem den auf diese Weise bestimmten Wert mit einem vorbestimmten Schwellenwert vergleicht, der je nach dem verwendeten Verhältnistyp gewählt ist, so dass die Werte dieses Verhältnisses, die größer bzw. kleiner als dieser Schwellenwert sind, repräsentativ für die Werte sind, die bei Patienten mit einem anerkannten Prostata-Adenokarzinom beobachtet wurden, und so dass umgekehrt die Werte dieses Verhältnisses, die kleiner bzw. größer als dieser Schwellenwert sind, repräsentativ für die Werte sind, die bei Patienten beobachtet wurden, die dafür anerkannt sind, an keinem Prostata-Adenokarzinom zu leiden, und man aus dem Ergebnis dieses Vergleichs entweder auf das Vorliegen oder auf das Nichtvorliegen eines Prostata-Adenokarzinoms schließt.

18. Methode nach Anspruch 16 oder 17, bei der man:
- die Menge an aktivem freien PSA und die Menge an inaktivem freien PSA,
wobei das inaktive freie PSA aus gespaltenem inaktiven freien PSA und der Zymmogenform des PSA besteht, in einer bei diesem Patienten entnommenen Probe bestimmt,
- ggf. die Menge einer anderen PSA-Form als dem aktiven freien PSA und dem inaktiven freien PSA, wobei dieses inaktive freie PSA aus gespaltenem inaktiven freien PSA und der Zymogenform des PSA besteht, in einer bei demselben Patienten entnommenen Probe derselben Natur bestimmt, die aus gesamtem freien PSA, Gesamt-PSA und komplexiertem PSA ausgewählt ist,
- den Wert eines der folgenden Verhältnisse bestimmt:
Verhältnis der Menge an inaktivem freien PSA, wobei dieses inaktive freie PSA aus gespaltenem inaktiven freien PSA und der Zymogenform des PSA besteht, zur Menge an aktivem freien PSA,
Verhältnis der Menge an inaktivem freien PSA, wobei dieses inaktive freie PSA aus gespaltenem inaktiven freien PSA und der Zymogenform des PSA besteht, zur Menge an gesamtem freien PSA,
Verhältnis der Menge an inaktivem freien PSA, wobei dieses inaktive freie PSA aus gespaltenem inaktiven freien PSA und der Zymogenform des PSA besteht, mit der Menge an Gesamt-PSA,
Verhältnis der Menge an inaktivem freien PSA, wobei dieses inaktive freie PSA aus gespaltenem inaktiven freien PSA und der Zymogenform des PSA besteht, zur Menge an komplexiertem PSA,
oder die Kehrwerte dieser Verhältnisse oder Kombinationen dieser Verhältnisse,
- den auf diese Weise bestimmten Wert des Verhältnisses mit einem vorbestimmten Schwellenwert vergleicht, der je nach dem verwendeten Verhältnistyp gewählt ist, so dass die Werte dieses Verhältnisses, die größer bzw. kleiner als der Schwellenwert sind, repräsentativ für die Werte sind, die bei Patienten mit einem anerkannten Prostata-Adenokarzinom beobachtet wurden, und so dass umgekehrt die Werte dieses Verhältnisses, die kleiner bzw. größer als dieser Schwellenwert sind, repräsentativ für die Werte sind, die bei Patienten beobachtet wurden, die dafür anerkannt sind, dass sie an keinem Prostata-Adenokarzinom leiden, und man aus dem Ergebnis dieses Vergleichs entweder auf das Vorliegen oder Nichtvorliegen eines Prostata-Adenokarzinoms schließt.

19. Diagnostikkit, das die Diagnostizierung eines Prostata-Adenokarzinoms bei einem Patienten mit Verdacht auf dieses Adenokarzinom gestattet oder die Differenzierung zwischen einer gutartigen Prostataerkrankung oder einem Prostata-Adenokarzinom bei einem Patienten mit Verdacht auf eine dieser Krankheiten gestattet, wobei dieses Kit umfasst:
- Antikörper, wie sie in Anspruch 1 oder 2 definiert sind, zur Dosierung des inaktiven freien PSA, wobei dieses inaktive freie PSA aus gespaltenem inaktiven freien PSA und der Zymogenform des PSA besteht, in einer biologischen Probe und/oder
- Mittel zur Dosierung des aktiven freien PSA in einer biologichen Probe derselben Natur und ggf.
- Mittel zur Dosierung einer anderen PSA-Form als inaktives freies PSA, wobei dieses inaktive freie PSA aus gespaltenem inaktiven freien PSA und der Zymogenform des PSA besteht, und als aktives freies PSA in einer biologichen Probe derselben Natur, ausgewählt aus gesamtem freien PSA, Gesamt-PSA und komplexiertem PSA.

20. Kit nach Anspruch 19, bei dem diese Mittel Antikörper sind.
